# EUROPEAN PATENT APPLICATION

(11) **EP 3 640 325 A1**
(43) Date of publication of application: **22.04.2020**
(21) Application number: 19200735.9
(22) Date of filing: 23.06.2014
(51) Int. Cl.: C12N 5/071, C12N 5/0783

(54) **METHODS OF EXPANDING T CELLS**

(30) Priority: 24.06.2013 US 201361838685 P
(62) Divisional of application: 14817301.6
(71) Applicant: Celgene Corporation, Summit, NJ 07901 (US)
(72) Inventor: ABBOT, Stewart, Warren, NJ 07059 (US); FOSS, Willard, San Diego, CA 92130 (US); DANIEL, Thomas, Lajolla, CA 92192 (US)
(74) Representative: Jones Day

(57) **Abstract**

Provided herein is an *in vitro* method of inducing a population of immune cells to proliferate, comprising contacting the immune cells with an antibody against CD3 and a ligand of a costimulatory molecule on said immune cells, such that said immune cells are caused to proliferate; wherein said antibody against CD3 and said ligand are comprised within a composition; wherein said composition comprises a liquid phase that comprises a hydrophobic molecule, or an albumin; wherein said ligand of said costimulatory molecule is an antibody against CD28, an antibody against CD9, B7-1, B7-2, or a CD28-binding fragment of B7-1 or B7-2; wherein said antibody against CD3 and said ligand are not immobilized on the surface of said composition; and wherein said antibody against CD3 and said ligand of a costimulatory molecule are each covalently bound to said hydrophobic molecule or complexed with said albumin.

## Description

This application claims priority to U.S. Provisional Patent Application No. 61/838,685, filed June 24, 2013, the disclosure of which is incorporated herein by reference in its entirety.

### 1. FIELD

Provided herein are methods in the field of cell culture, specifically of culture and expansion of immune cells, e.g., T lymphocytes.

### 2. BACKGROUND

T lymphocytes (T cells) may be cultured and expanded by a number of methods. One current method of expanding T cells utilizes an antibody against a primary signaling moiety on the T cells, e.g., an anti-CD3 antibody, in combination with an antibody against a costimulatory moiety on the T cells, e.g., and antibody against CD28, where both antibodies are immobilized on a solid surface, e.g., a bead or culture dish surface. However, there exists a need in the art for other methods of expanding immune cells. The present disclosure provides such other methods.

### 3. SUMMARY

Provided herein are methods of expanding a population of immune cells, e.g., T cells, using at least one primary stimulatory ligand (also referred to herein as a primary ligand) and at least one ligand of a costimulatory molecule (also referred to herein as a costimulatory ligand).

In one aspect, provided herein is a method of inducing a population of immune cells to proliferate, comprising contacting the immune cells with a primary ligand, e.g., a ligand of a T cell receptor (TCR)/CD3 complex on said immune cells, and a costimulatory ligand on said immune cells, such that said immune cells are caused to proliferate, wherein said ligands are comprised within a composition, wherein said composition comprises a liquid phase or multi-construct phase, and wherein said ligands are not immobilized on the surface of said composition. In certain embodiments, the composition comprises a polymer, a hydrogel, an albumin, and/or a hydrophobic molecule. In certain embodiments, the composition is in the form of a bead, e.g., is substantially spherical. The composition is not an adjuvant (e.g., is not an aluminum salt, calcium phosphate hydroxide, beryllium, an oil (e.g., mineral oil or paraffin oil) *per se,* a detergent *per se,* squalene, thimerosol (ethylmercury), Freund's complete adjuvant, Freund's incomplete adjuvant), or the like.

In certain embodiments, the primary ligand, e.g., ligand of said TCR/CD3 complex, is a macromolecular polypeptide binding agent. In a specific embodiment, the ligand is an antibody against CD3 (anti-CD3). The antibody may be polyclonal or monoclonal. The antibody may be from a hybridoma clone, and may be a humanized form of a non-human antibody. In a specific embodiment, the monoclonal antibody is OKT3 (Muronomab-CD3) or G19-4, or a CD3-binding portion thereof. The binding agent may also be a mimotope.

In certain embodiments, the costimulatory ligand is a ligand for CD28. In certain embodiments, the costimulatory ligand is a macromolecular polypeptide binding agent against CD28. The costimulatory ligand can be, for example, an antibody against CD28 (anti-CD28). In certain embodiments, the costimulatory ligand is B7-1 or B7-2 or a CD28-binding fragment thereof. The costimulatory ligand can be, for example, an antibody against CD137 (anti-CD137). In another embodiment, the costimulatory ligand is an antibody against CD9 (anti-CD9). In a specific embodiment, the anti-CD9 antibody is ES5.2D8. The antibodies may be polyclonal or monoclonal. The antibodies may be from a hybridoma clone, and may be a humanized form of a non-human antibody.

In a specific embodiment of any of the embodiments presented herein, the primary and costimulatory ligands are anti-CD3 and anti-CD28, respectively, e.g., the ligand of said TCR/CD3 complex is anti-CD3 and the ligand of a costimulatory molecule on said immune cells is anti-CD28. In a specific embodiment of any of the embodiments presented herein, the ligands are anti-CD3 and anti-CD137 (anti-4-1BB), e.g., the ligand of said TCR/CD3 complex is anti-CD3 and the ligand of a costimulatory molecule on said immune cells is anti-CD137.

In certain embodiments, the composition comprises a hydrogel. The hydrogel may be a self-assembling hydrogel, e.g., a hydrogel comprising a polyglutamate chain and vitamin E. In a specific embodiment, one or both of said ligands are contained within a mesh of said hydrogel.

In certain other embodiments, the composition comprises an oil, lipid or fluorocarbon, wherein said oil, lipid or fluorocarbon is a liquid, e.g., exists as a liquid in aqueous solution. In specific embodiments, said oil, lipid or fluorocarbon is present in the composition in the form of nanometer- or micrometer-scale droplets. In a specific embodiment, said ligands are present in said composition in or on the surface of said microdroplets and micelles.

In certain embodiments, the composition comprising the ligands comprises albumin. In a specific embodiment, albumin is complexed with a liquid hydrophobic molecule, e.g., an oil, a lipid, or a fluorocarbon. In a specific embodiment, a portion of, or substantially all of, said albumin is present on the surface of a droplet of said hydrophobic molecule. In a more specific embodiment, a portion of, or substantially all of, said albumin and said ligands are present on the surface of a droplet of said hydrophobic molecule. In a specific embodiment, the albumin is non-native albumin, e.g., the albumin has a structure other than that as albumin exists in serum. In a more specific embodiment, said albumin is denatured.

In another embodiment of the method, at least one of, or both of, said primary and/or costimulatory ligands is conjugated to a hydrophobic molecule. In a specific embodiment, the primary ligand, e.g., CD3 ligand, is conjugated to a hydrophobic molecule. In another specific embodiment, the costimulatory ligand is conjugated to a hydrophobic molecule. In a more specific embodiment, the costimulatory ligand is a ligand of CD28. In a more specific embodiment, said costimulatory and primary ligands are anti-CD28 and anti-CD3, respectively, and both of said ligands are conjugated to a hydrophobic molecule. In another more specific embodiment, said ligands respectively conjugated to a hydrophobic molecule are anti-CD3 and one or B7-1 or B7-2.

In another embodiment of the method, at least one of, or both of, said primary and/or costimulatory ligands is conjugated via a linker molecule to a hydrophobic molecule. In a specific embodiment, the primary ligand, e.g., CD3 ligand, is conjugated to an anti-CD3-specific antibody to a hydrophobic molecule. In a more specific embodiment, the costimulatory ligand is a ligand of CD28 and the linker is an anti-CD3-specific antibody.

In other embodiments, said composition comprises a hydrophobic molecule, wherein neither of said primary or costimulatory ligands is conjugated to said hydrophobic molecule. For example, in a specific embodiment, said composition comprises a hydrophobic molecule, wherein said ligands are anti-CD28 and anti-CD3, and neither of said anti-CD28 or said anti-CD3 is conjugated to said hydrophobic molecule. In another specific embodiment, said composition comprises a hydrophobic molecule, wherein said ligands are anti-CD3 and one of B7-1 or B7-2, and wherein neither of said anti-CD3 or said one of B7-1 or B7-2 is conjugated to said hydrophobic molecule.

In any of the above embodiments, said hydrophobic molecule self-aggregates in aqueous solution to form a micelle. In various embodiments, said hydrophobic molecule is a lipid, an aliphatic chain, or a fluorocarbon (e.g., a perfluorocarbon).

In embodiments in which either or both of the primary and/or costimulatory ligands are complexed with albumin, said albumin may be human serum albumin.

In a specific embodiment of any of the embodiments of the method provided herein, the composition comprises a liquid phase or multi-phase system that is largely immiscible in aqueous media between 2°C and 44°C. In a specific embodiment, the composition presents as a colloid or an emulsion with aqueous media between 2°C and 44°C upon homogenization.

In certain specific embodiments of any of the embodiments herein, said immune cells are T cells, e.g., in non-limiting examples, CD4+ T cells, CD8+ T cells, or Treg cells. In certain specific embodiments, the T cells are CD45RO-, CD45RA+, CCR7+, CD62L+, IL-2Rβ-, and CD95-. In certain other specific embodiments, the T cells are CD45RO-, CD45RA+, CCR7+, CD62L+, IL-2Rβ+, and CD95+. In certain other specific embodiments, the T cells are CD45RO+, CD45RA-, CCR7+, CD62L+, IL-2Rβ+, and CD95+. In certain other specific embodiments, the T cells are CD45RO+, CD45RA-, CCR7-, CD62L-, IL-2Rβ+, and CD95+. In certain other specific embodiments, the T cells are CD45RO+, CD45RA+, CCR7-, CD62L-, IL-2Rβ+, and CD95+. In certain specific embodiments, the T cells are CD3+, CD8+, CD27+, CD28+, CD45RO-, CD45RA+, CCR7+, CD62L+, IL-2Rβ+, IL-7R+, and CD95-. In other specific embodiments, the T cells express one or more of CCR7, CXCR4, and L-selectin. In other specific embodiments, the T cells do not express CCR7 and L-selectin. In other specific embodiments, the T cells are CCR9+, α4β7+, CCR7+, and either L-selectin^{low} or L-selectin-. In other specific embodiments, the T cells are CCD4+, CCD10+, LFA-1+, and α4β1+. In a specific embodiment, the T cells are Central Memory T cells, or about 20%, 25%, 30%, 35%, 40%, 45%, 50%, 55%, 60%, 65%, 70%, 75%, 80%, 85%, 90%, or 95% of the T cells are Central Memory T cells. In another specific embodiment, the T cells are Central Memory stem T cells, or about 20%, 25%, 30%, 35%, 40%, 45%, 50%, 55%, 60%, 65%, 70%, 75%, 80%, 85%, 90%, or 95% of the T cells are Central Memory stem T cells. In certain embodiments, the T cells have been contacted with TWS119 during culture. In other embodiments, the T cells have been contacted with exogenous IL-2, IL-7, IL-21, and/or IL-15 during culture. In any of the embodiments described herein, the immune cells are additionally contacted with a phorbol ester, e.g., 12-0-tetradecanoylphorbol-13-acetate (TPA; also referred to as phorbol-12-myristate-13-acetate (PMA)).

In a specific embodiment of any of the embodiments of the method provided herein, the immune cells are expanded from about 100-fold to about 100,000-fold.

### 4. DETAILED DESCRIPTION

Provided herein is a method of expanding immune cells, e.g., T lymphocytes (T cells), using ligands of a primary signaling protein (primary ligands) and of a costimulatory domain (costimulatory ligands) on the immune cells, wherein the ligands are not both immobilized on a surface, e.g., a solid surface.

Thus, provided herein is a method of inducing a population of immune cells to proliferate, comprising contacting the immune cells with a ligand of a primary signaling domain or protein (primary ligand) on said immune cells, and a ligand of a costimulatory domain (costimulatory ligand) on said immune cells, such that said immune cells are caused to proliferate, wherein said ligands are comprised within a composition, wherein said composition comprises a liquid phase or multi-construct phase that is not an adjuvant, and wherein said ligands are not immobilized on the surface of said composition. As used herein, "ligand" indicates any macromolecule, e.g., protein or portion thereof, that is capable of binding, e.g., a primary signaling domain or protein of an immune cell or a costimulatory domain on an immune cell, and includes, e.g., antibodies or binding portions thereof, receptors or binding portions thereof, or artificially- or naturally-occurring variants of such biomolecules.

The primary signaling domain can be, for example, a protein that is part of the T cell receptor (TCR)/CD3 complex, e.g., CD3y, CD3δ or CD3ε, or a functional portion thereof. In certain embodiments, the ligand of said TCR/CD3 complex is a macromolecular polypeptide binding agent. In a specific embodiment, the ligand is an antibody against CD3 (anti-CD3), e.g., anti-CD3y, anti-CD3δ or anti-CD3ε. The antibody may be polyclonal or monoclonal. The antibody may be from a hybridoma clone, and may be a humanized form of a non-human antibody. In a specific embodiment, the monoclonal antibody is OKT3 or G19-4 or a CD3-binding portion thereof.

The primary signaling domain may also be CD2. Where this is the case, the ligand may be, for example, and antibody against CD2.

In certain embodiments, the costimulatory ligand is a ligand for CD28. In certain embodiments, the costimulatory ligand is a macromolecular polypeptide binding agent against CD28. The costimulatory ligand can be, for example, an antibody against CD28 (anti-CD28). Such an antibody may be, for example, monoclonal antibody 9.3 or EX5.3D10 (ATCC Deposit No. HB11373). In certain embodiments, the costimulatory ligand is B7-1 or B7-2 or a CD28-binding fragment thereof. The costimulatory ligand can be, for example, an antibody against CD137 (anti-CD 137). The ligand of said costimulatory molecule can be, for example, an antibody against 4-1BB (anti-4-1BB). In another embodiment, the costimulatory ligand is an antibody against CD9 (anti-CD9). In a specific embodiment, the anti-CD9 antibody is ES5.2D8. The antibodies may be polyclonal or monoclonal. The antibodies may be from a hybridoma clone, and may be a humanized form of a non-human antibody. In another embodiment, the costimulatory ligand is an antibody against CD2 (anti-CD27). In another embodiment, the costimulatory ligand is an antibody against ICOS (anti-ICOS). In another embodiment, the costimulatory ligand is an antibody against CD40L (anti-CD40L). In another embodiment, the costimulatory ligand is an antibody against OX40 (anti-OX40).

In a specific embodiment of any of the embodiments presented herein, the ligands are anti-CD3 and anti-CD28, e.g., the ligand of said TCR/CD3 complex is anti-CD3 and the ligand of a costimulatory molecule on said immune cells is anti-CD28. In a specific embodiment of any of the embodiments presented herein, the ligands are anti-CD3 and anti-CD137 (anti-4-1BB), e.g., the ligand of said TCR/CD3 complex is anti-CD3 and the ligand of a costimulatory molecule on said immune cells is anti-CD137.

In certain embodiments, provided herein is a method of inducing a population of immune cells to proliferate, comprising contacting the immune cells with a composition comprising active agents Zap70, akt, wav-1 and diacylglycerol (DAG), wherein said composition comprises a liquid phase or multi-construct phase that is not an adjuvant. In specific embodiments, said active agents are not immobilized on a surface, e.g., the surface of said composition. In other specific embodiments, one or more, or all, of said active agents are immobilized on a surface, e.g., the surface of said composition.

The composition, including either or both of the primary and costimulatory ligands, can be any shape that orients at least a portion of the ligands such that the ligands are capable of being perceived by a portion of the immune cells and cause the immune cells to proliferate. In certain embodiments, the composition comprises ligands to a primary signaling domain on an immune cell, wherein at least 5%, 10%, 15%, 20%, 25%, 30%, 35%, 40%, 45%, 50%, 55%, 60%, 65%, 70%, 75%, 80%, 85%, 90%, or 95% of the ligands are in an orientation such that said ligands can bind to said primary signaling domain on the immune cell, e.g., as measurable using, e.g., an anti-idiotype antibody, or fluorophore-labeled ligand, in flow cytometry. In certain embodiments, the composition comprises ligands to a costimulatory molecule on an immune cell, wherein at least 5%, 10%, 15%, 20%, 25%, 30%, 35%, 40%, 45%, 50%, 55%, 60%, 65%, 70%, 75%, 80%, 85%, 90%, or 95% of the ligands are in an orientation such that said ligands can bind to said costimulatory molecule on the immune cell, e.g., as measurable using, e.g., an anti-idiotype antibody, or fluorophore-labeled ligand, in flow cytometry. In certain embodiments, the composition is in the form of a bead, e.g., is substantially spherical. In certain embodiments, the composition comprises ligands to a primary signaling domain and a costimulatory molecule, wherein both ligands are randomly distributed throughout said composition. In other embodiments, the composition comprises ligands to a primary signaling domain and a costimulatory molecule, wherein both ligands are fluidly present in the surface of the composition, e.g., at a hydrophobic/hydrophilic interface.

### 4.1. Compositions Comprising Ligands and a Polymers

The composition comprising the primary and costimulatory ligands can further comprise a polymer that acts, e.g., as a binding agent to hold the primary and co-stimulatory ligands together in a micelle. Such polymers can be, for example, amphiphilic polymers or hydrogels.

In certain embodiments, the composition comprises a hydrogel. Any hydrogel that is suitable for cell culture may be used. In preferred embodiments, the composition comprises a self-assembling hydrogel. Self-assembling hydrogels that may be used in the methods provided herein include, for example, cross-linked dextran microspheres modified with L- or D-oligolactate chains (see, e.g., van Tomme et al., "Macroscopic hydrogels by self-assembly of oligolactate-grafted dextran microspheres," Biomacromolecules 9(1):158-165 (2008)); loading of the ligands onto the hydrogels can be accomplished by mixing the microspheres with a solution comprising the ligands. The microspheres can also comprise, for example, polyvinyl alcohol (PVA) and poly(vinyl pyrrolidone) (PVP).

While any self-assembling hydrogel may be used, a preferred embodiment incorporates use of an amphiphilic polymer that comprises a polyglutamate chain and a vitamin E moiety, e.g., MEDUSA® hydrogel from Flamel Technologies (Venessieux, France). Compositions are formed by combining the hydrated hydrogel with the first and second ligands such that microspheres of the ligand-containing hydrogel are formed. Other amphiphilic polymers that may be used include, e.g., amphiphilic polymers comprising polyethylene glycol as the hydrophilic moiety and a combination of cetyltrimethylammonium bromide (CTAB) and sodium perflouorohexanate (FC(5) or FC(7)) chains as the hydrophobic moiety (see Kang et al., Biomacromolecules 4(2):360-365 (2003); poly(2-acrylanidohexadecylsulfonic acid) (PAMC₁₈S); polyethylele glycol as the hydrophilic moiety and a combination of desaminotyrosyl-tyrosine ethyl ester (DTE) and desaminotyrosyl-tyrosine (DT) as the hydrophobic moiety (see Kahn et al., J. Funct. Biomater. 3(4):745-759 (2012).

### 4.2. Compositions Comprising Ligands and a Hydrophobic Compound

In certain embodiments, the composition comprises a hydrophobic compound, e.g., a hydrophobic moiety or a hydrophobic liquid. The composition may comprise a hydrophobic compound and the ligands, wherein the ligands are not covalently bound to the hydrophobic compound. The composition may comprise a hydrophobic compound and the ligands, wherein one or both of the ligands are respectively covalently bound to the hydrophobic compound.

Hydrophobic liquids, when emulsified, naturally form micelles suitable for bearing the ligands. Suitable hydrophobic compounds for forming micelles include oils or liquid fluorocarbons, e.g., perfluorooctylbromide, perflubron, and the like. In preferred embodiments, the hydrophobic compound does not act to denature the ligands.

In certain embodiments, one or both ligands may be covalently bound to a hydrophobic moiety to form a fusion protein or an immunoconjugate; pluralities of such fusion proteins or immunoconjugates may naturally form micelles as the hydrophobic moieties self-aggregate in aqueous solution. Ligands such as antibodies may be bound, e.g., to a hydrophobic compound comprising, e.g., polyethylene glycol (PEG) and a diacyl lipid. In specific embodiments, the diacyl lipid is phosphatidylethanolamine (PE). In a specific embodiment, PEG-PE is conjugated to a ligand through a *p*-nitrophenylcarbonyl (pNP) linker, e.g., pNP-PEG-PE-ligand conjugates. Immunomicelles comprising pNP-PEG-PE-ligand immunoconjugates can be produced from ligands bearing a free NH₂ group by mixing the ligands with pNP-PEG-PE at pH 8.0 to 9.5; *see* Torchlin et al., "Immunomicelles: Targeted pharmaceutical carriers for poorly soluble drugs," PNAS 100(10):6039-6044 (2003).

### 4.3. Compositions Comprising Ligands and Albumin

In certain embodiments, the composition comprises albumin, e.g., human serum albumin. The albumin may be native albumin (e.g., albumin that has not been denatured), or may be denatured albumin. The denatured albumin may be produced from native albumin by, e.g., subjecting the native albumin to mechanical shearing forces, e.g., by sonication, or by passage of a solution comprising the native albumin through a jet spray or similar device.

The albumin and ligands may be mixed together, e.g., in solution, e.g., by stirring, agitation, vortexing, or other methods that thoroughly combine two solutions.

The albumin may first be combined with a hydrophobic compound prior to combining with the ligands. Preferably, the albumen and hydrophobic compound are combined in such a way as to cause the formation of microspheres of the hydrophobic compound with the hydrophobic compound on the interior of the microspheres and the albumin on the exterior of the microspheres. Methods for producing such albumen-coated microspheres are described, e.g., in International Patent Application No. PCT/US2012/037137, and in U.S. Patent No. 5,498,421, the disclosure of which is hereby incorporated by reference in its entirety. In one embodiment, the albumin is first complexed with a hydrophobic compound, e.g., an oil, e.g., soybean oil, by overlaying a IX volume of aqueous 1%-5% albumin solution with a 1X-4X volume of oil, bringing the temperature to 10°C -45°C , e.g., 20°C, and sonicating for 30 seconds. In another embodiment, the albumin is first complexed with a hydrophobic compound, e.g., a liquid fluorocarbon, e.g., perfluorooctylbromide, by overlaying a IX volume of aqueous 1%-5% albumin solution with a 1X-4X volume of the fluorocarbon, bringing the temperature to 10°C - 45°C, e.g., 20°C, and sonicating for 30 seconds. In each case, the resulting emulsions may be diluted with a saline solution.

Once such microspheres are produced, the ligands may be complexed with the microspheres by stirring, agitation or vortexing. The size of the ligand/albumin complexes can be changed by varying the amount of ligand used to complex with the albumin/hydrophobic compound microspheres. The ligands may be used, e.g., at about 0.5, 1.0, 1.5, 2.0, 2.5, 3.0, 3.5, 4.0, 4.5, 5.0, 6.0, 7.0, 8.0, 9.0, 10.0, 11.0, 12.0, 13.0, 14.0 or 15.0 mg/mL, plus or minus about 20%. In certain embodiments, in the aggregate, the ligands complexed with albumin, or albumin/hydrophobic compound microspheres, retain the ability to bind their respective targets.

### 4.4. T Lymphocytes

In preferred embodiments of any of the embodiments herein, the immune cells expanded according to the methods provided herein are T cells (also referred to as T lymphocytes). In non-limiting examples, the T cells are CD4+ T cells, CD8+ T cells, or Treg cells. The T cells may be used at any stage along the lineage of T cell development from T cell precursors.

T cells for use in the methods described herein may be obtained from a subject, or may be obtained from an existing culture of T cells. T cells that are obtained from a subject can be obtained from, e.g., mammals, e.g., humans, canines, felines, rodents, and transgenic species thereof. The T cells can be obtained from, e.g., peripheral blood, placental blood, umbilical cord blood, bone marrow, lymph node tissue, spleen tissue, tumors, or the like. Cells from peripheral blood can be separated, e.g., by leukopheresis. T cells may be obtained from peripheral blood leukocytes by optionally lysing red blood cells, and separating peripheral blood leukocytes from monocytes by, for example, centrifugation through a PERCOLL™ gradient. Specific subpopulations of T cells, such as CD28+, CD4+, CD8+, CD28RA+, and CD28RO+ T cells, or any of the other T cell populations described below, may be isolated from the leukocytes by positive and/or negative selection techniques. A preferred method for either positive or negative selection is cell sorting via negative magnetic immunoadherence utilizing a one or more monoclonal antibodies directed to cell surface markers present on the cells to be positively selected for, or negatively selected against. Appropriate combinations of antibodies for either positive or negative selection will be apparent to those of skill in the art.

T cells can be cultured under conditions appropriate for T cell culture, e.g., including appropriate media (e.g., Minimal Essential Media or RPMI Media 1640) which may contain factors necessary for proliferation and viability, including animal serum (e.g., fetal bovine serum) and antibiotics (e.g., penicillin streptomycin). The T cells are typically maintained under conditions necessary to support growth, for example an appropriate temperature (e.g., 37°C) and atmosphere (e.g., air plus 5% CO₂).

The T cells, cultured and expanded using the methods provided herein, may be, for example, CD45RO-, CD45RA+, CCR7+, CD62L+, IL-2Rβ-, and CD95-. A population of cells may be cultured and expanded according to the methods provided herein, wherein at least 20%, 25%, 30%, 35%, 40%, 45%, 50%, 55%, 60%, 65%, 70%, 75%, 80%, 85%, 90% or 95% of the cells in said population are CD45RO-, CD45RA+, CCR7+, CD62L+, IL-2Rβ-, and CD95-T cells.

In certain other specific embodiments, the T cells are CD45RO-, CD45RA+, CCR7+, CD62L+, IL-2Rβ+, and CD95+. A population of cells may be cultured and expanded according to the methods provided herein, wherein at least 20%, 25%, 30%, 35%, 40%, 45%, 50%, 55%, 60%, 65%, 70%, 75%, 80%, 85%, 90% or 95% of the cells in said population are CD45RO-, CD45RA+, CCR7+, CD62L+, IL-2Rβ+, and CD95+ T cells.

In certain other specific embodiments, the T cells are CD45RO+, CD45RA-, CCR7+, CD62L+, IL-2Rβ+, and CD95+. A population of cells may be cultured and expanded according to the methods provided herein, wherein at least 20%, 25%, 30%, 35%, 40%, 45%, 50%, 55%, 60%, 65%, 70%, 75%, 80%, 85%, 90% or 95% of the cells in said population are CD45RO+, CD45RA-, CCR7+, CD62L+, IL-2Rβ+, and CD95+ T cells.

In certain other specific embodiments, the T cells are CD45RO+, CD45RA-, CCR7-, CD62L-, IL-2Rβ+, and CD95+. A population of cells may be cultured and expanded according to the methods provided herein, wherein at least 20%, 25%, 30%, 35%, 40%, 45%, 50%, 55%, 60%, 65%, 70%, 75%, 80%, 85%, 90% or 95% of the cells in said population are CD45RO+, CD45RA-, CCR7-, CD62L-, IL-2Rβ+, and CD95+ T cells.

In certain other specific embodiments, the T cells are CD45RO+, CD45RA+, CCR7-, CD62L-, IL-2Rβ+, and CD95+. A population of cells may be cultured and expanded according to the methods provided herein, wherein at least 20%, 25%, 30%, 35%, 40%, 45%, 50%, 55%, 60%, 65%, 70%, 75%, 80%, 85%, 90% or 95% of the cells in said population are CD45RO+, CD45RA+, CCR7-, CD62L-, IL-2Rβ+, and CD95+ T cells.

In certain specific embodiments, the T cells are CD3+, CD8+, CD27+, CD28+, CD45RO-, CD45RA+, CCR7+, CD62L+, IL-2Rβ+, IL-7R+, and CD95-. A population of cells may be cultured and expanded according to the methods provided herein, wherein at least 20%, 25%, 30%, 35%, 40%, 45%, 50%, 55%, 60%, 65%, 70%, 75%, 80%, 85%, 90% or 95% of the cells in said population are CD3+, CD8+, CD27+, CD28+, CD45RO-, CD45RA+, CCR7+, CD62L+, IL-2Rβ+, IL-7R+, and CD95- T cells. In other specific embodiments, the T cells express one or more of CCR7, CXCR4, and L-selectin.

In other specific embodiments, the T cells do not express CCR7 and L-selectin. A population of cells may be cultured and expanded according to the methods provided herein, wherein at least 20%, 25%, 30%, 35%, 40%, 45%, 50%, 55%, 60%, 65%, 70%, 75%, 80%, 85%, 90% or 95% of the cells in said population do not express CCR7 and L-selectin.

In other specific embodiments, the T cells are CCR9+, α4β7+, CCR7+, and either L-selectin^{low} or L-selectin-. A population of cells may be cultured and expanded according to the methods provided herein, wherein at least 20%, 25%, 30%, 35%, 40%, 45%, 50%, 55%, 60%, 65%, 70%, 75%, 80%, 85%, 90% or 95% of the cells in said population are T cells that are CCR9+, α4β7+, CCR7+, and either L-selectin^{low} or L-selectin-.

In other specific embodiments, the T cells are CCD4+, CCD10+, LFA-1+, and α4β1+. A population of cells may be cultured and expanded according to the methods provided herein, wherein at least 20%, 25%, 30%, 35%, 40%, 45%, 50%, 55%, 60%, 65%, 70%, 75%, 80%, 85%, 90% or 95% of the cells in said population are T cells that are CCD4+, CCD10+, LFA-1+, and α4β1+.

In a specific embodiment, the T cells are Central Memory T cells, or about 20%, 25%, 30%, 35%, 40%, 45%, 50%, 55%, 60%, 65%, 70%, 75%, 80%, 85%, 90%, or 95% of the T cells are Central Memory T cells. In another specific embodiment, the T cells are Central Memory stem T cells, or about 20%, 25%, 30%, 35%, 40%, 45%, 50%, 55%, 60%, 65%, 70%, 75%, 80%, 85%, 90%, or 95% of the T cells are Central Memory stem T cells.

In certain embodiments, the T cells have been contacted with an inhibitor of glycogen synthase kinase-3β (GSK3β) during at least a portion of, or during the whole time, of culture according to the methods provided herein. In a specific embodiment, the inhibitor of GSK3β is TWS119.

In other embodiments, the T cells have been contacted with exogenous IL-2, IL-7, IL-21, and/or IL-15 during at least a portion of, or during the whole time, of expansion in culture according to the methods provided herein, that is, in certain embodiments of the method of expanding immune cells, e.g., T cells, described herein, the method comprises contacting the immune cells with exogenous IL-2, IL-7, IL-21, and/or IL-15 during at least a portion of, or during the whole time, of expansion.

In any of the embodiments described herein, the immune cells are additionally contacted with a nonspecific stimulator of T cell proliferation, e.g., phorbol ester, e.g., 12-0-tetradecanoylphorbol-13-acetate (TPA; also referred to as phorbol-12-myristate-13-acetate (PMA)) during at least a portion of, or during the whole time, of culture according to the methods provided herein.

In any of the embodiments described herein, expansion of the immune cells using the primary signaling ligands and costimulatory ligands results in a cell population comprising about 10⁷ to about 10⁸, about 10⁸ to about 10⁹, about 10⁹ to 10¹⁰, about 10¹⁰ to about 10¹¹, about 10¹¹ to about 10¹² or about 10¹² to about 10¹³ said immune cells.

### 4.5. Expansion of T Cells

The T cells and the ligand-containing compositions described above may be placed into contact with each other, e.g., in a culture dish of vessel appropriate for the culture of T cells. In embodiments in which the composition is present in the form of microspheres, the ratio of T cells to microspheres may be anywhere between 10:1 to 1:10,0000, e.g., in non-limiting examples, 1:10, 1:20, 1:30, 1:40, 1:50, 1:60, 1:70, 1:80, 1:90, 1:100, 1:200, 1:300, 1:400, 1:500, 1:600, 1:700, 1:800, 1:900, 1:1000 or 1:10,000. Expansion of T cells may proceed for, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days or more. The degree of expansion may be analyzed at one or more time points during expansion by, e.g., counting a sample of cells with a cell counter, flow cytometry, or the like. The rate of expansion may be determined, e.g., by examining the size or measuring the volume of the T cells, such as with a Coulter Counter. A resting T cell has a mean diameter of about 6.8 microns. Following the initial activation and stimulation and in the presence of the stimulating ligand, the T cell mean diameter is expected to increase to over 12 microns by day 4 and begin to decrease by about day 6.

For maintenance of long term stimulation of a population of T cells following initial activation and stimulation, T cells can be separated from the ligand-containing composition, e.g., microcarriers, from the activating stimulus (e.g., the anti-CD3 antibody) after a period of exposure, while maintaining contact between the T cells and the costimulatory ligand throughout the culture term. The rate of T cell proliferation may monitored periodically (e.g., daily) by, for example, examining the size or measuring the volume of the T cells, such as with a Coulter Counter, as above; after the mean cell diameter decreases to approximately 8 microns, the T cells may be reactivated and restimulated to induce further proliferation. Alternatively, the rate of T cell proliferation and time for T cell restimulation can be monitored by assaying for the presence of cell surface molecules, such as B7-1, B7-2, which are induced on activated T cells; a decrease in the level of B7-1 expression, relative to the level of expression following an initial or previous stimulation or the level of expression in an unstimulated cell, may indicate the time for restimulation. Alternatively, the rate of T cell proliferation and time for T cell restimulation can be monitored by assaying for nutrition consumption, for example, glucose consumption.

Induction of long term stimulation of a population of CD4+ or CD8+ T cells may be accomplished by reactivation and restimulation of the T cells with a primary ligand and a costimulatory ligand, e.g., a anti-CD3 antibody and an anti-CD28 antibody or monoclonal antibody ES5.2D8, one or more times to produce a population of CD4+ or CD8+ cells increased in number from about 10- to about 1,000-fold the original T cell population. Culture of T cells can be accomplished, either in the presence or absence of one or both of the primary ligand and/or costimulatory ligand, according to art-recognized methods. T cells can be cultured in serum-free medium, or medium comprising, e.g., human serum, fetal bovine serum, calf serum, or the like. Medium in which the T cells are cultured, e.g., expanded, may contain one or more cytokines, e.g., interleukin-s (IL-2), IL-4, IL-7, IL-10, IL-15, and/or IL-21. Such cytokines may be added once during culture, or e.g. every 1, 2, 3, 4, or 5 days.

### 4.6. Antibodies

Either or both of the primary ligand or costimulatory ligand may be antibodies. Antibodies may be obtained from commercial sources, e.g., the American Type Culture Collection, ABCAM®, or the like. Antibodies may also be produced by standard techniques. Antibodies useful in the methods of expansion of immune cells provided herein may be of any class, e.g., IgG, IgM, IgE, IgA, or IgD, or artificial variants thereof having at least 2 epitope binding sites.

Antibodies may be produced using an immunogen, optionally presented as a conjugate linked to a carrier, typically at about 10 micrograms to about 500 milligrams per immunization dose, preferably about 50 micrograms to about 50 milligrams per dose, depending upon the animal immunized. An immunization preparation can also include an adjuvant as part of the diluents, e.g., Freund's adjuvant (CFA), incomplete Freund's adjuvant (IFA), or alum. Such adjuvants are well known in the art, and are available commercially from several sources.

The immunogen can comprise, e.g., native proteins (for example, native Cd3, native CD28, native CD137, or the like), or synthetic peptides based on such native proteins. Both soluble and membrane bound forms of the protein or peptide fragments are suitable for use as immunogens. The purified protein can also be covalently or noncovalently modified with non-proteinaceous materials such as lipids or carbohydrates to enhance immunogenecity or solubility. Whole proteins may be purified from cellular sources for use as immunogens. Where peptides are to be used as immunogens, sequences of the native proteins, and of immunogenic portions thereof, are readily available, e.g., in the GenBank or SwissProt public sequence databases.

A purified protein or peptide, e.g., a CD28 protein or peptide fragment thereof, may be conjugated to a carrier that is immunogenic in animals, for example, albumins, serum proteins (e.g., globulins and lipoproteins), polyamino acids, bovine serum albumin, rabbit serum albumin, thyroglobulin, keyhole limpet hemocyanin, egg ovalbumin and bovine gamma-globulins. Chemical cross-linking agents that are known to those skilled in the art may be used for such conjugation, e.g., succinimidyl 4-(N-maleimidomethyl)cyclohexane-1-carboxylate (SMCC), m-Maleimidobenzoyl-N-hydroxysuccinimide ester (MBS); N-succinimidyl(4-iodoacetyl)aminobenzoate (SIAB), succinimidyl 4-(p-maleimidophenyl)butyrate (SMPB), 1-ethyl-3-(3-dimethylaminopropyl)carbodiimide hydrochloride (EDC); 4-succinimidyloxycarbonyl-a-methyl-a-(2-pyridyldithio)-tolune (SMPT), N-succinimidyl 3-(2-pyridyldithio)propionate (SPDP), succinimidyl 6-[3-(2-pyridyldithio)propionate]hexanoate (LC-SPDP), or the like.

Antibodies to either or both of the primary ligand or costimulatory ligand may be or comprise polycolonal antibodies. Polyclonal antibodies can generally be raised in animals by multiple subcutaneous (sc) or intraperitoneal (ip) injections of an appropriate immunogen, such as the extracellular domain of the protein, and an adjuvant, e.g., as described in Lindsten, T. et al. (1993) J. Immunol. 151:3489-3499, and by other known techniques.

Antibodies to either or both of the primary ligand or costimulatory ligand may be or comprise monoclonal antibodies. Monoclonal antibodies may be prepared by, e.g., the hybridoma technique originally described by Kohler and Milstein (1975, Nature 256:495-497), the human B cell hybridoma technique (Kozbor et al. (1983) Immunol Today 4:72), EBV-hybridoma technique (Cole et al. (1985), Monoclonal Antibodies and Cancer Therapy, Alan R. Liss, Inc., pp. 77-96), trioma techniques, or phage display techniques (Marks et al. (1992) J. Biol Chem 16007-16010). Monoclonal antibody compositions of the invention can also be produced by other methods well known to those skilled in the art of recombinant DNA technology, for example, the "combinatorial antibody display" method, developed to identify and isolate antibody fragments having a particular antigen specificity, and can be utilized to produce monoclonal antibodies. Methods of combinatorial display are known in the art; see e.g., Sastry et al. (1989) PNAS 86:5728; Huse et al. (1989) Science 246:1275; and Orlandi et al. (1989) PNAS86:3833).

### 5. EXAMPLES

### 5.1. Example 1: Expansion of T Cells Using Anti-CD3+Anti-CD28 and a Hydrogel

T cells are obtained from 250 mL of the peripheral blood of a subject. A lymphocyte fraction is obtained by leukopheresis, and red blood cells in the fraction are lysed. A T cell population is obtained by positive selection for cells that are CD4+, CD45RA+ and CD62L+ using antibodies from Beckman-Coulter or LifeSpan BioSciences, conjugated to magnetic beads, followed by negative selection in the resulting population of cells using antibodies to CD45RO, IL-2Rβ and CD95 from LifeSpan BioSciences, conjugated to magnetic beads. A population of CD45RO-, CD45RA+, CCR7+, CD62L+, IL-2Rβ-, and CD95-T cells is obtained.

Antibodies directed to CD3 and CD28, both from ABCAM®, are selected as primary ligand and costimulatory ligand, respectively. The antibodies are combined in solution with the MEDUSA® hydrogel, a self-assembling hydrogel comprising a polyglutamate chain and vitamin E, under conditions that produce microspheres of about 100 to about 500 micrometers. Routine varying of the relative amounts of the antibodies and hydrogel enables correct sizing.

1 x 10⁷ of the T cells are cultured in the presence of the hydrogel/ligand microspheres at a ratio of about 1:10 cells:microspheres. Culture is continued for 28 days, with optional cell counting every 7 days. The protocol is expected to generate between 1 x 10¹⁰ and 1 x 10¹¹ activated T cells.

### 5.2. Expansion of T Cells Using Anti-CD3+Anti-CD28 and an Oil Plus Albumin

Anti-CD3 and -CD28 antibodies are selected as ligands, and T cells are prepared, as in Example 1.

A IX volume of aqueous 5% albumin solution is overlaid with a 2X volume of oil, and the temperature of the albumin/oil is brought to 20°C in a water bath. The albumin solution and oil are sonicated for 30 seconds to emulsify. The emulsion is then diluted 1:5 with a physiologically-acceptable saline solution.

1 x 10⁷ of the T cells in 10 ml culture medium are cultured in the presence of the ligand-containing emulsion at about 1:1 v:v. Culture is continued for 28 days, with optional cell counting every 7 days. The protocol is expected to generate between 1 x 10¹⁰ and 1 x 10¹¹ activated T cells.

### 5.3. Expansion of T Cells Using Anti-CD3+Anti-CD28 and a Perfluorocarbon Plus Albumin

Anti-CD3 and -CD28 antibodies are selected as ligands, and T cells are prepared, as in Example 1.

A IX volume of aqueous 5% albumin solution is overlaid with a 2X volume of perfluorooctylbromide, and the temperature of the albumin solution/perfluorooctylbromide is brought to 20°C in a water bath. The albumin solution and perfluorooctylbromide are then sonicated for 30 seconds to emulsify. The emulsion is then diluted 1:5 with a physiologically-acceptable saline solution.

1 x 10⁷ of the T cells in 10 ml culture medium are cultured in the presence of the ligand-containing emulsion at about 1:1 v:v. Culture is continued for 28 days, with optional cell counting every 7 days. The protocol is expected to generate between 1 x 10¹⁰ and 1 x 10¹¹ activated T cells.

### 5.4. Expansion of T Cells Using Anti-CD3+Anti-CD28 and a Perfluorocarbon

Anti-CD3 and -CD28 antibodies are selected as ligands, and T cells are prepared, as in Example 1. A IX volume of an aqueous solution containing both the anti-CD3 and anti-CD28 antibodies is overlaid with a 2X volume of perfluorooctylbromide, and the temperature of the albumin solution/perfluorooctylbromide is brought to 20°C in a water bath. The albumin solution and perfluorooctylbromide are then sonicated for 30 seconds to emulsify. The emulsion is then diluted 1:5 with a physiologically-acceptable saline solution.

1 x 10⁷ of the T cells in 10 ml culture medium are cultured in the presence of the ligand-containing emulsion at about 1:1 v:v. Culture is continued for 28 days, with optional cell counting every 7 days. The protocol is expected to generate between 1 x 10¹⁰ and 1 x 10¹¹ activated T cells.

### 5.5. Expansion of T Cells Using Anti-CD3 and Anti-CD28 and an Oil

Anti-CD3 and -CD28 antibodies are selected as ligands, and T cells are prepared, as in Example 1. A IX volume of an aqueous solution containing both the anti-CD3 and anti-CD28 antibodies is overlaid with a 2X volume of soybean oil, and the temperature of the albumin solution/perfluorooctylbromide is brought to 20°C in a water bath. The albumin solution and oil are then sonicated for 30 seconds to emulsify. The emulsion is then diluted 1:5 with a physiologically-acceptable saline solution.

1 x 10⁷ of the T cells in 10 ml culture medium are cultured in the presence of the ligand-containing emulsion at about 1:1 v:v. Culture is continued for 28 days, with optional cell counting every 7 days. The protocol is expected to generate between 1 x 10¹⁰ and 1 x 10¹¹ activated T cells.

All publications and patent applications cited in this specification are hereby incorporated by reference. Although the foregoing has been described in some detail by way of illustration and example for purposes of clarity of understanding, it will be readily apparent to those of ordinary skill in the art in light of the teachings herein that certain changes and modifications may be made thereto without departing from the spirit or scope of the appended claims.

**Also** disclosed are the following items:
1. A method of inducing a population of immune cells to proliferate, comprising contacting the immune cells with a ligand of a T cell receptor (TCR)/CD3 complex on said immune cells, and a ligand of a costimulatory molecule on said immune cells, such that said immune cells are caused to proliferate, wherein said ligands are comprised within a composition, wherein said composition comprises a liquid phase or multi-construct phase, and wherein said ligands are not immobilized on the surface of said composition.
2. The method of item 1, wherein said composition comprises a hydrogel, an albumin, or a hydrophobic molecule.
3. The method of item 1, wherein said composition is in the form of a bead.
4. The method of item 1, wherein said composition is substantially spherical.
5. The method of item 1, wherein said ligand of said TCR/CD3 complex is a macromolecular polypeptide binding agent.
6. The method of item 5, wherein said ligand is an antibody against CD3 (anti-CD3).
7. The method of item 6, wherein said antibody is polyclonal.
8. The method of item 6, wherein said antibody is monoclonal.
9. The method of item 6, wherein said antibody is the monoclonal antibody is OKT3 or G19-4 or a CD3-binding portion thereof.
10. The method of item 1, wherein said ligand of a costimulatory molecule is a ligand for CD28.
11. The method of item 1, wherein said ligand of said costimulatory molecule is a macromolecular polypeptide binding agent against CD28.
12. The method of item 1, wherein said ligand of said costimulatory molecule is an antibody against CD28 (anti-CD28).
13. The method of item 12, wherein said antibody is polyclonal.
14. The method of item 12, wherein said antibody is monoclonal.
15. The method of item 1, wherein said ligand of said costimulatory molecule is an antibody against CD9.
16. The method of item 15, wherein said antibody is polyclonal.
17. The method of item 15, wherein said antibody is monoclonal.
18. The method of item 15, wherein said antibody is ES5.2D8.
19. The method of item 1, wherein said ligand of said costimulatory molecule is B7-1 or B7-2 or a CD28-binding fragment thereof.
20. The method of item 1, wherein said composition comprises a hydrogel.
21. The method of item 20, wherein said hydrogel is a self-assembling hydrogel.
22. The method of item 20, wherein said hydrogel comprises a polyglutamate chain and vitamin E.
23. The method of any of items 20-22 wherein said ligands are contained within a mesh of said hydrogel.
24. The method of item 1, wherein the composition comprises an oil, lipid or fluorocarbon, wherein said oil, lipid or fluorocarbon is a liquid.
25. The method of item 24, wherein said oil, lipid or fluorocarbon is present in the composition in the form of nanometer- or micrometer-scale droplets.
26. The method of item 24, wherein said ligands are present in said composition on the surface of said microdroplets.
27. The method of item 26, wherein said ligands are anti-CD3 and anti-CD28.
28. The method of item 1, wherein the composition comprises albumin.
29. The method of item 28, wherein said albumin is complexed with a liquid hydrophobic molecule.
30. The method of item 29, wherein said hydrophobic molecule is an oil, a lipid, or a fluorocarbon.
31. The method of item 29 or item 30, wherein said albumin is present on the surface of a droplet of said hydrophobic molecule.
32. The method of item 29 or item 30, wherein said albumin and said ligands are present on the surface of a droplet of said hydrophobic molecule.
33. The method of any of items 28-32, wherein the albumin is non-native albumin.
34. The method of any of items 28-32, wherein said albumin is denatured.
35. The method of item 1, wherein at least one of said ligands is conjugated to a hydrophobic molecule.
36. The method of item 1, wherein the CD3 ligand is conjugated to a hydrophobic molecule.
37. The method of item 1, wherein said ligand to a costimulatory molecule is conjugated to a hydrophobic molecule.
38. The method of item 1, wherein the ligand is a ligand of CD28.
39. The method of item 1, wherein each of said ligands is respectively conjugated to a hydrophobic molecule.
40. The method of item 39, wherein said ligands are anti-CD28 and anti-CD3.
41. The method of item 39, wherein said ligands are anti-CD3 and one or B7-1 or B7-2.
42. The method of item 1, wherein said composition comprises a hydrophobic molecule, wherein neither of said ligands is conjugated to said hydrophobic molecule.
43. The method of item 39, wherein said composition comprises a hydrophobic molecule, wherein said ligands are anti-CD28 and anti-CD3, and neither of said anti-CD28 or said anti-CD3 is conjugated to said hydrophobic molecule.
44. The method of item 39, wherein said composition comprises a hydrophobic molecule, wherein said ligands are anti-CD3 and one of B7-1 or B7-2, and neither of said anti-CD3 or said one of B7-1 or B7-2 is conjugated to said hydrophobic molecule.
45. The method of any of items 35-44, wherein said hydrophobic molecule self-aggregates in aqueous solution to form a micelle.
46. The method of any of items 35-44, wherein said hydrophobic molecule is a lipid.
47. The method of any of items 35-44, wherein said hydrophobic molecule is an aliphatic chain.
48. The method of any of items 35-44, wherein said hydrophobic molecule is a fluorocarbon.
49. The method of any of items 2-48, wherein said albumin is human serum albumin.
50. The method of any of items 1-49, wherein said immune cells are T cells.
51. The method of item 50, wherein said T cells are CD4+ T cells.
52. The method of item 50, wherein said T cells are CD8+ T cells.
53. The method of item 50, wherein said T cells are Treg cells.
54. The method of any of items 1-53, wherein the immune cells are expanded about 100-fold to about 100,000-fold.
55. The method of any of items 1-54, wherein the composition comprises a liquid phase or multi-phase system that is largely immiscible with aqueous media between 2°C and 44°C.
56. The method of item 1, wherein the composition presents as a colloid or an emulsion with aqueous media between 2°C and 44°C upon homogenization.
57. The method of any of items 1-56, additionally comprising contacting the immune cells with interleukin-2 (IL-2).
58. The method of any of items 1-57, additionally comprising contacting the immune cells with a phorbol ester.

## Claims

1. An *in vitro* method of inducing a population of immune cells to proliferate, comprising contacting the immune cells with an antibody against CD3 and a ligand of a costimulatory molecule on said immune cells, such that said immune cells are caused to proliferate; wherein said antibody against CD3 and said ligand are comprised within a composition; wherein said composition comprises a liquid phase that comprises a hydrophobic molecule, or an albumin; wherein said ligand of said costimulatory molecule is an antibody against CD28, an antibody against CD9, B7-1, B7-2, or a CD28-binding fragment of B7-1 or B7-2; wherein said antibody against CD3 and said ligand are not immobilized on the surface of said composition; and wherein said antibody against CD3 and said ligand of a costimulatory molecule are each covalently bound to said hydrophobic molecule or complexed with said albumin.

2. The method of claim 1, wherein said ligand of said costimulatory molecule is an antibody against CD28.

3. The method of claim 1 or 2, wherein said immune cells are T cells.

4. The method of claim 3, wherein said T cells are CD4+ T cells, CD8+ T cells, or Treg cells.

5. The method of any one of claims 1 to 4, wherein the method additionally comprises contacting the immune cells with interleukin-2 (IL-2), or with a phorbol ester.

6. The method of any one of claims 1 to 5, wherein said composition is in the form of a bead.

7. The method of any one of claims 1 to 6, wherein said hydrophobic molecule is an oil, lipid or fluorocarbon, wherein said oil, lipid or fluorocarbon is a liquid.

8. The method of claim 7, wherein said oil, lipid or fluorocarbon is present in the composition in the form of nanometer- or micrometer-scale droplets.

9. The method of any one of claims 1 to 8, wherein said hydrophobic molecule self-aggregates in aqueous solution to form a micelle.
